# EUROPEAN PATENT SPECIFICATION

(11) **EP 0 953 630 B1**
(45) Date of publication and mention of the grant of the patent: **08.09.2004**
(21) Application number: 99106122.7
(22) Date of filing: 23.06.1992
(51) Int. Cl.: C11C 3/06, A61K 47/44, A61K 9/107, A61K 38/13

(54) **Transesterification products of corn oil and glycerol and their use in pharmaceutical compositions**
Umesterungsprodukte aus Maisöl und Glycerol und ihre Verwendung in pharmaceutischen Zusammensetzungen
Produits de transestérification entre l'huile de mais et le glycérol et leur utilisation dans des compositions pharmaceutiques

(30) Priority: 27.06.1991 GB 9113872
(43) Date of publication of application: 03.11.1999
(62) Divisional of application: 92810479.3
(73) Proprietor: Novartis AG, 4056 Basel (CH)
(72) Inventor: Hauer, Birgit, 77933 Lahr (DE); Meinzer, Armin, 79426 Buggingen (DE); Posanski, Ulrich, / (DE); Vonderscher, Jacky, 68400 Riedisheim (FR)
(74) Representative: Grubb, Philip William

(56) References cited:
- EP-A- 0 378 893
- WO-A-91/08676
- GB-A- 2 228 198

## Description

This application is derived from EP 92810479.3. The present invention relates to transesterified corn oil products and compositions comprising the same.

A pharmaceutical composition is disclosed in EP 92810479.3 comprising a cyclosporin as active ingredient in a carrier medium comprising:
1) 1,2-propylene glycol;
2) a mixed mono-, di-, tri-glyceride; and
3) a hydrophilic surfactant.

The term "pharmaceutical composition" as used herein and in the accompanying claims is to be understood as defining compositions of which the individual components or ingredients are themselves pharmaceutically acceptable, e.g. where oral administration is foreseen, suitable or acceptable for oral application.

Components (2) in the compositions disclosed in EP 92810479.3 comprise mixtures of C₁₂₋₂₀ fatty acid mono-, di- and tri-glycerides, especially C₁₆₋₁₈ fatty acid mono-, di- and triglycerides. The fatty acid component of said mixed mono-, di- and tri-glycerides may comprise both saturated and unsaturated fatty acid residues. Preferably however they will predominantly be comprised of unsaturated fatty acid residues in particular, C₁₈ unsaturated fatty acid residues for example linolenic, linoleic and oleic acid residues. Suitably component (2) will comprise at least 60%, preferably at least 75%, more preferably 85% or more by weight C₁₈ unsaturated fatty acid, e.g. linolenic, linoleic and oleic acid mono-, di- and tri-glycerides. Suitably they will comprise less than 20%, e.g. ca. 15% or 10% by weight or less, saturated fatty acid, e.g. palmitic and stearic acid mono-, di- and tri-glycerides.

Components (2) in the above-mentioned compositions will preferably be predominantly comprised of mono- and di-glycerides, e.g. comprise at least 50%, more preferably at least 70%, e.g. 75%, 80%, 85% by weight or more, mono- and di-glycerides, based on the total weight of component (2).

Components (2) in the compositions of the invention will suitably comprise from about 25 to about 50%, preferably from about 30 to about 40%, e.g. 35 to 40%, monoglycerides, based on the total weight of component (2).

Components (2) in the composition of the invention will suitably comprise from about 30 to about 60%, preferably from about 40 to about 55%, e.g. about 48 to 50%, di-glycerides, based on the total weight of component (2).

Components (2) in the compositions of the invention will suitably comprise at least 5%, e.g. from about 7.5 to about 15%, e.g. 9 to 12%, by weight of triglycerides.

Components (2) in the compositions of the invention may be prepared by admixture of individual mono-, di- or tri-glycerides in appropriate relative proportion. Conveniently however they will comprise transesterification products of vegetable oils, for example almond oil, ground nut oil, olive oil, peach oil, palm oil or, preferably, corn oil, sunflower oil or safflower oil and most preferably corn oil, with glycerol.

In another aspect, this invention provides
a trans-esterification product of corn oil and glycerol comprising predominately of linoleic acid and oleic acid mono-, di- and tri-glycerides, and having a free glycerol content less than 10% by weight, wherein the product has been treated so as to
a) reduce the saturated fatty acid component content of mono-, di- and tri-glycerides; and to
b) enhance the unsaturated fatty acid component content of mono-, di- and tri-glycerides so that the linoleic acid and oleic acid mono-, di- and tri-glyceride content is in total 85% by weight or more of the whole composition, and
the product has a total palmitic acid and stearic acid content of mono-, di- and tri-glycerides of less than 10% by weight.

Such transesterification products are generally obtained by heating of the selected vegetable oil with glycerol, at high temperature in the presence of an appropriate catalyst under an inert atmosphere with continuous agitation, e.g. in a stainless steel reactor, to effect trans-esterification or glycerolysis. In addition to their mono-, di- and tri-glyceride components, such transestrification products will also generally comprise minor amounts of free glycerol. The amount of free glycerol present in components (2) for use in the compositions of the invention will preferably be less than 10%, more preferably less than 5%, most preferably ca. 1 or 2% by weight based on the total weight of free glycerol plus mono-, di- and tri-glycerides.

Preferably some of the glycerol is first removed e.g. by distillation (to give a "substantially glycerol free batch"), when soft gelatine capsules are to be made.

Preferably components (2) have a low saturated fatty acid content. Components (2) meeting these requirements may, for example be obtained from commercially available products, e.g. obtained therefrom by methods such as separative techniques as known in the art, e.g. freezing procedures coupled with separative techniques, e.g. centrifugation, to remove the saturated fatty acid components/enhance the unsaturated fatty acid component content. Typically the total saturated fatty acid component content will be <15%, e.g. <10%, or <5% by weight based on the total weight of component (2). A reduction of the content of saturated fatty acid component in the mono-glyceride fraction of components (2) may be observed after the separative technique.

Components (2) thus preferably contain lesser quantities of saturated fatty acids (e.g. palmitic and stearic acids) and relatively greater quantities of unsaturated fatty acids (e.g. oleic and linoleic acids) than for the starting material.

Typical preferred components (2) may according to the preferred embodiment of this invention contain:

| | |
|---|---|
| 32-36% | mono-glycerides, |
| 45-55% | di-glycerides and |
| 12-20% | tri-glycerides |

by weight based on the total weight of component (2).

Further preferred characteristics include the following:

| Fatty acid content as determined as the methyl ester by chromatography | |
|---|---|
| Methyl linoleate | 53-63% |
| Methyl oleate | 24-34% |
| Methyl linolenate | 0- 3% |
| Methyl arachate | 0- 3% |
| Methyl palmitate | 6-12% |
| Methyl stearate | 1- 3% |
| | |
| Relative Density | 0.94-0.96 |
| Hydroxyl Value | 140-210 |
| Iodine Value | 110-120 |
| Peroxide Value | <4.0 |
| Free Glycerol | <1.0 |
| Acid value max. | ca. 2 |
| Saponification no. ca. | 150-185 |

Components (2) complying with the above outlined features are referred to hereafter as "refined glycerol-transesterified corn oils". Freshly prepared components (2) according to the preferred embodiments are of clear appearance and stay clear at storage temperature of 20°C - 25°C for more than 20 days.

The "refined glycerol-transesterified corn oils" have especially been proposed for the preparation of the compositions disclosed in EP 92810479.3. They may also have uses for the solubilization of other active agents and have the advantage of remaining stable, e.g. clear, for a long time.

The following example illustrates the present invention.

### EXAMPLE 1:

Preparation of "refined glycerol-transesterified corn oil".

Substantially-glycerol free glycerol-transesterified corn oil (if necessary after heating to give a clear mixture) is slowly cooled to a temperature of +20°C and kept at this temperature for one night. In a first-step centrifugation, at an acceleration of 12 000 G and a flow rate of 103 kg/h in a continuous flow centrifuge, a liquid phase (62 kg/h) and a sediment-containing phase (41 kg/h) are obtained. The liquid phase is slowly cooled to +8°C and kept at this temperature for one night. In a second-step centrifugation at an acceleration of 12 000 G and a flow rate of 112 kg/h a liquid phase (76.2 kg/h) and a sediment-containing phase (35.8 kg/h) are obtained. The liquid phase is "refined glycerol-transesterified corn oil". Alternatively an improved product may be obtained by effecting the centrifugation in three steps, e.g. at +20°C, +10°C and +5°C.

The process is characterised by a slight percentage reduction in the mono-glyceride component in the refined glycerol transesterified corn oil as compared to the starting material (e.g. 35.6% compared to 38.3%).

A typical analytical comparison between the sediment and clear solution is as follows:

| | **Compound** | **Sediment (X)** | **Clear Solution (%)** |
|---|---|---|---|
| 1. | Mono palmitate | 19.1 | 3.4 |
| 2. | Mono linoleate + Mono oleate | 23.4 | 27.0 |
| 3. | Mono stearate | 5.7 | <2 |
| 4. | Dilinoleate + Dioleate | 35.4 | 44.7 |
| 5. | Other di-glycerides | 7.7 | 10.4 |
| 6. | Tri-glycerides | 8.7 | 12.5 |

Typical contents of components in the refined product obtained from these preparations are listed in the folloving Table:

| **COMPOSITION OF COMPONENTS (% v/v)** | |
|---|---|
| Components | refined glycerol-trans-esterified corn oil |
| Glycerides: mono | 33.3 |
| di | 52.1 |
| tri | 14.6 |

| Fatty acids: | |
|---|---|
| palmitic acid (C16) | 7.8 |
| stearic acid (C18) | 1.7 |
| oleic acid (C18:1) | 31.6 |
| linoleic acid (C18:2) | 57.7 |
| Glycerol content | <1% |

## Claims

1. A trans-esterification product of com oil and glycerol comprising predominately of linoleic acid and oleic acid mono-, di- and tri-glycerides, and having a free glycerol content less than 10% by weight, wherein the product has been treated so as to
a) reduce the saturated fatty acid component content of mono-, di- and tri-glycerides; and to
b) enhance the unsaturated fatty acid component content of mono-, di- and tri-glycerides so that the linoleic acid and oleic acid mono-, di- and tri-glyceride content is in total 85% by weight or more of the whole composition, and
the product has a total palmitic acid and stearic acid content of mono-, di- and tri-glycerides of less than 10% by weight.

2. A product as claimed in claim 1 having 30% to 40% by weight of mono-glycerides.

3. A product as claimed in claim 1 or claim 2 having 45% to 55% by weight of di-glycerides.

4. A product as claimed in any preceding claim having from about 7.5 to about 15 % by weight of tri-glycerides.

5. A product as claimed in any preceding claim having a free glycerol content of less than 5% by weight.

6. A product as claimed in any preceding claim having a free glycerol content of less than 2% by weight.

7. A transesterification product of corn oil and glycerol having a saturated fatty acid content of mono-, di-, and tri-glycerides, and having a free glycerol content less than 10% by weight, which product comprises
i) from about 25% to about 50% by weight of mono-glycerides, from about 30% to about 60% by weight of di-glycerides, and at least 5% by weight of tri-glycerides; and
ii) a linoleic acid, oleic acid and linolenic acid mono-, di- and tri-glyceride content of at least 85% by weight;
wherein the total palmitic acid and stearic acid content of mono-, di-, and tri-glycerides is less than 10% by

8. A product as claimed in claim 7 having 30% to 40% by weight of mono-glycerides.

9. A product as claimed in claim 7 or claim 8 having 45% to 55% by weight of di-glycerides.

10. A product as claimed in any one of claims 7 to 9 having from about 7.5 to about 15 % by weight of tri-glycerides.

11. A product as claimed in any one of claims 7 to 10 having a free glycerol content of less than 5% by weight.

12. A product as claimed in any one of claims 7 to 11 having a free glycerol content of less than 2% by weight.

13. A process for obtaining a refined glycerol-transesterified corn oil according to any preceding claim, comprising heating of corn oil with glycerol at high temperature in the presence of a suitable catalyst under an inert atmosphere with continuous agitation to effect glycerol-transesterification, and refining said product by freezing procedures coupled with separative techniques.

14. A pharmaceutical composition comprising a product as claimed in any of claims 1 to 6.

15. Use of a product as claimed in any of claims 1 to 6 in a pharmaceutical composition.

16. A pharmaceutical composition comprising a product as claimed in any of claims 7 to 12.

17. Use of a product as claimed in any of claims 7 to 12 in a pharmaceutical composition.

## Patentansprüche

1. Umesterungsprodukt aus Maisöl und Glycerin, das vorwiegend Linolsäure- und Ölsäuremono-, -di- und -triglyceride enthält und einen Gehalt an freiem Glycerin von weniger als 10 Gew.-% aufweist, wobei dieses Produkt zwecks
a) Erniedrigung des gesättigten Fettsäurekomponentengehalts der Mono-, Di- und Triglyceride hiervon und
b) Erhöhung des ungesättigten Fettsäurekomponentengehalts der Mono-, Di- und Triglyceride hiervon einer solchen Behandlung unterzogen worden ist, dass
der Linolsäure- und Ölsäuremono-, -di- und -triglyceridgehalt insgesamt 85 Gew.-% oder mehr der Gesamtzusammensetzung ausmacht und
das Produkt einen gesamten Palmitinsäure- und Stearinsäuregehalt der Mono-, Di- und Triglyceride hiervon von weniger als 10 Gew.-% aufweist.

2. Produkt nach Anspruch 1, das 30 bis 40 Gew.-% Monoglyceride enthält.

3. Produkt nach Anspruch 1 oder 2, das 45 bis 55 Gew.-% Diglyceride enthält.

4. Produkt nach einem der vorhergehenden Ansprüche, das etwa 7,5 bis etwa 15 Gew.-% Triglyceride enthält.

5. Produkt nach einem der vorhergehenden Ansprüche, das einen Gehalt an freiem Glycerin von weniger als 5 Gew.-% aufweist.

6. Produkt nach einem der vorhergehenden Ansprüche, das einen Gehalt an freiem Glycerin von weniger als 2 Gew.-% aufweist.

7. Umesterungsprodukt aus Maisöl und Glycerin mit einem gesättigten Fettsäuregehalt der Mono-, Di- und Triglyceride und einem Gehalt an freiem Glycerin von weniger als 10 Gew.-%, wobei dieses Produkt umfasst
i) etwa 25 Gew.-% bis etwa 50 Gew.-% Monoglyceride, etwa 30 Gew.-% bis etwa 60 Gew.-% Diglyceride und wenigstens 5 Gew.-% Triglyceride und
ii) einen Linolsäure-, Ölsäure- und Linolensäuremono-, -di- und -triglyceridgehalt von wenigstens 85 Gew.-%, wobei
der gesamte Palmitinsäure- und Stearinsäuregehalt der Mono-, Di- und Triglyceride hiervon weniger als 10 Gew.-% beträgt.

8. Produkt nach Anspruch 7, das 30 bis 40 Gew.-% Monoglyceride enthält.

9. Produkt nach Anspruch 7 oder 8, das 45 bis 55 Gew.-% Diglyceride enthält.

10. Produkt nach einem der Ansprüche 7 bis 9, das etwa 7,5 bis etwa 15 Gew.-% Triglyceride enthält.

11. Produkt nach einem der Ansprüche 7 bis 10, das einen Gehalt an freiem Glycerin von weniger als 5 Gew.-% aufweist.

12. Produkt nach einem der Ansprüche 7 bis 11, das einen Gehalt an freiem Glycerin von weniger als 2 Gew.-% aufweist.

13. Verfahren zum Erhalt eines raffinierten und mit Glycerin umgeesterten Maisöls nach einem der vorhergehenden Ansprüche durch Erhitzung von Maisöl mit Glycerin auf eine hohe Temperatur in Gegenwart eines geeigneten Katalysators unter einer Inertatmosphäre und kontinuierlicher Durchmischung zwecks Bewirkung einer Glycerinumesterung und durch Raffination des erhaltenen Produkts durch Gefrierverfahren gekoppelt mit Separationstechniken.

14. Pharmazeutische Zusammensetzung, umfassend ein Produkt gemäß einem der Ansprüche 1 bis 6.

15. Verwendung eines Produkts gemäß einem der Ansprüche 1 bis 6 in einer pharmazeutischen Zusammensetzung.

16. Pharmazeutische Zusammensetzung umfassend ein Produkt nach einem der Ansprüche 7 bis 12.

17. Verwendung eines Produkts nach einem der Ansprüche 7 bis 12 in einer pharmazeutischen Zusammensetzung.

## Revendications

1. Produit de transestérification d'huile de maïs et de glycérol comprenant en majorité des mono-, di- et tri-glycérides d'acide linoléique et d'acide oléique et présentant une teneur en glycérol libre inférieure à 10% en poids, **caractérisé en ce que** ledit produit est traité de façon à
a) réduire la teneur en mono-, di- et tri-glycérides de composant acide gras saturé ; et à
b) accroître la teneur en mono-, di- et tri-glycérides de composant acide gras insaturé,
de sorte que la teneur en mono-, di- et tri-glycérides d'acide linoléique et d'acide oléique soit égale à 85% en poids, ou plus, de toute la composition, et
que le produit présente une teneur totale en mono-, di- et tri-glycérides d'acide palmitique et d'acide stéarique de moins de 10% en poids.

2. Produit selon la revendication 1, ayant de 30% à 40% en poids de mono-glycérides.

3. Produit selon la revendication 1 ou 2 ayant de 45% à 55% en poids de di-glycérides.

4. Produit selon l'une quelconque des revendications précédentes ayant d'environ 7,5% à environ 15% en poids de triglycérides.

5. Produit selon l'une quelconque des revendications précédentes ayant une teneur en glycérol libre inférieure à 5% en poids.

6. Produit selon l'une quelconque des revendications précédentes ayant une teneur en glycérol libre inférieure à 2% en poids.

7. Produit de transestérification d'huile de maïs et de glycérol ayant une teneur en mono-, di- et tri-glycérides d'acide gras saturé et présentant une teneur en glycérol libre inférieure à 10% en poids, ledit produit comprennant :
i) d'environ 25% à environ 50% en poids de mono-glycérides, d'environ 30% à environ 60% en poids de di-glycérides et au moins 5% en poids de tri-glycérides et,
ii) une teneur en mono-, di- et tri-glycérides d'acide linoléique, d'acide oléique et d'acide linolénique d'au moins 85% en poids,
**caractérisé en ce que** la teneur totale en mono-, di- et tri-glycérides d'acide palmitique et d'acide stéarique est inférieure à 10% en poids.

8. Produit selon la revendication 7 ayant de 30% à 40% en poids de mono-glycérides.

9. Produit selon la revendication 7 ou 8 ayant de 45% à 55% en poids de di-glycérides.

10. Produit selon l'une quelconque des revendications 7 à 9 ayant d'environ 7,5% à environ 15% en poids de tri-glycérides.

11. Produit selon l'une quelconque des revendications 7 à 10 ayant une teneur en glycérol libre inférieure à 5% en poids.

12. Produit selon l'une quelconque des revendications 7 à 11 ayant une teneur en glycérol libre inférieure à 2% en poids.

13. Procédé pour obtenir une huile de maïs transestérifiée par le glycérol et de qualité raffinée selon l'une quelconque des revendications précédentes, comprenant le chauffage de l'huile de maïs avec du glycérol et ce, à haute température, en présence d'un catalyseur adéquat, sous atmosphère inerte et sous agitation continue afin de réaliser la transestérification avec le glycérol ainsi que le raffinage dudit produit à l'aide de procédés de congélation couplés à des techniques de séparation.

14. Composition pharmaceutique comprenant un produit selon l'une quelconque des revendications 1 à 6.

15. Utilisation d'un produit selon l'une quelconque des revendications 1 à 6 dans une composition pharmaceutique.

16. Composition pharmaceutique comprenant un produit selon l'une quelconque des revendications 7 à 12.

17. Utilisation d'un produit selon l'une des revendications 7 à 12 dans une composition pharmaceutique.
